# EUROPÄISCHE PATENTSCHRIFT

(11) **EP 0 885 225 B1**
(45) Veröffentlichungstag und Bekanntmachung des Hinweises auf die Patenterteilung: **23.01.2002**
(21) Anmeldenummer: 97905050.7
(22) Anmeldetag: 19.02.1997
(51) Int. Cl.: C07D 487/04, A01N 43/90

(54) **TETRAHYDROPYRIMIDIN-DERIVATE**
TETRAHYDROPYRIMIDINE DERIVATIVES
DERIVES DE TETRAHYDROPYRIMIDINE

(30) Priorität: 04.03.1996 DE 19608243; 11.12.1996 DE 19651429
(43) Veröffentlichungstag der Anmeldung: 23.12.1998
(73) Patentinhaber: BAYER AG, 51368 Leverkusen (DE)
(72) Erfinder: KANELLAKOPULOS, Johannes, D-41542 Dormagen (DE); WOLLWEBER, Detlef, D-42133 Wuppertal (DE); ERDELEN, Christoph, D-42799 Leichlingen (DE)
(86) Internationale Anmeldenummer: EP9700775
(87) Internationale Veröffentlichungsnummer: WO9732878

(56) Entgegenhaltungen:
- EP-A- 0 247 477

## Beschreibung

Die vorliegende Erfindung betrifft Tetrahydropyrimidin-Derivate, Verfahren zu ihrer Herstellung und ihre Verwendung zur Bekämpfung von tierischen Schädlingen.

Es ist bereits bekannt, daß bestimmte bicyclische Pyrimidin-Derivate, wie insbesondere 6-substituierte 6,7-Dihydro-8-nitro-(5H)-1-(2-chlorpyridin-5-yl-methyl bzw. 2-chlor-1,3-thiazol-5-yl-methyl)-imidazolidino-[2,3-f]-pyrimidine, insektizide Eigenschaften aufweisen (vgl. z.B. EP-A-0 247 477). Die Wirksamkeit und Wirkungsbreite dieser Verbindungen ist jedoch, insbesondere bei niedrigen Aufwandmengen und Konzentrationen nicht immer voll zufriedenstellend.

Es wurden Tetrahydropyrimidin-Derivate der Formel (I) gefunden, 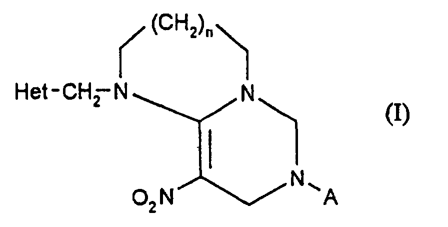 in welcher
- A: für eine Gruppierung (Ia) -(CH₂)ₘ-COXR¹ oder (Ib) 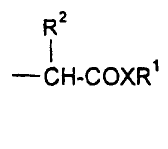 steht,
- Het: steht für jeweils gegebenenfalls einfach bis zweifach, gleich oder verschieden durch Fluor, Chlor oder Brom substituiertes Pyridyl und Thiazolyl.
- R¹: steht für gegebenenfalls einfach bis zweifach, gleich oder verschieden substituiertes Benzyl, wobei als Substituenten genannt seien:
Fluor, Chlor, Brom, Cyano, Nitro, Methyl, Ethyl, Methoxy, Methylthio, Trifluormethyl, Trifluormethoxy und Trifluormethylthio;
sowie für jeweils gegebenenfalls einfach bis zweifach, gleich oder verschieden durch Fluor, Chlor oder Brom substituiertes Pyridylmethyl und Thiazolylmethyl.
- R2: steht für C₁₋₄-Alkyl oder Benzyl;
- x: steht für Sauerstoff oder die -NH-Gruppierung.
- m: steht für ganze Zahlen von 1 bis 8.
- n: steht für die Zahlen 0 und 1.
Weiterhin wurde gefunden, daß man die Tetrahydropyrimidin-Derivate der Formel (I) erhält, wenn man
a) Nitromethylen-Derivate der Formel (II) 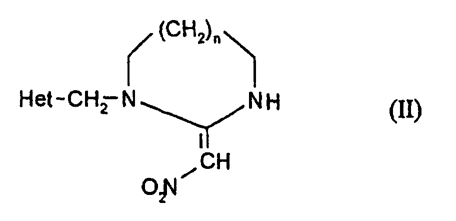 in welcher
   - Het und n: die oben angegebene Bedetung haben,
   mit Aminen der Formel (IIIa) oder (IIIb)

   H₂N-(CH₂)ₘ―CO-X-R¹ (IIIa)

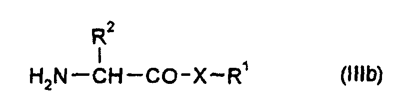

   in welcher
   - R¹, R², X und m: die oben angegebene Bedeutung haben,
   gegebenenfalls in Form ihrer Hydrohalogenide in Gegenwart von mindestens der zweifachen molaren Menge Formaldehyd, gegebenenfalls in Gegenwart eines sauren Katalysators und gegebenenfalls in Gegenwart eines Verdünnungsmittels sowie gegebenenfalls in Gegenwart einer Base umsetzt;
   oder
b) Nitropyrimidin-Derivate der Formel (IV) 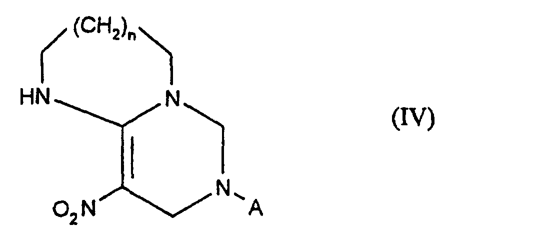
in welcher
- A und n: die oben angegebene Bedeutung haben,
mit Verbindungen der Formel (V)

Het-CH₂-L (V)

in welcher
- Het: die oben angegebene Bedeutung hat und
- L: für eine anionische Abgangsgruppe steht,
in Gegenwart einer Base und gegebenenfalls in Gegenwart eines Verdünnungsmittels umsetzt.

Schließlich wurde gefunden, daß die neuen Tetrahydropyrimidin-Derivate der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind.

Die erfindungsgemäßen Tetrahydropyrimidin-Derivate sind durch die Formel (I) allgemein definiert.

Die oben aufgeführten Restedefinitionen bzw. Erläuterungen gelten für die Endprodukte und für die Ausgangs- und Zwischenprodukte entsprechend. Diese Restedefinitionen können untereinander, beliebig kombiniert werden.

In den oben und nachstehend aufgeführten Restedefinitionen sind Kohlenwasserstoffreste, wie Alkyl oder Alkenyl - auch in Verbindungen mit Heteroatomen wie Alkoxy oder Alylthio - soweit möglich jeweils geradkettig oder verzweigt.

Bevorzugte erfindungsgemäße Verbindungen sind Stoffe der Formeln (Ic) bis (If): 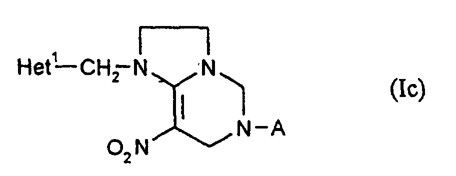 in welcher
- Het¹: für gegebenenfalls einfach bis zweifach, gleich oder verschieden durch Fluor, Chlor oder Brom substituiertes Pyridyl steht und
- A: die oben genannte Bedeutung hat. 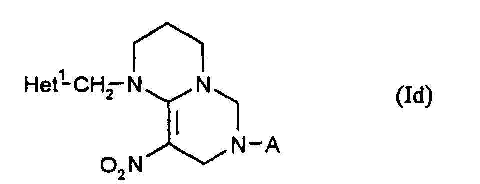 in welcher
- Het¹: für gegebenenfalls einfach bis zweifach, gleich oder verschieden durch Fluor, Chlor oder Brom substituiertes Pyridyl steht und
- A: die oben genannte Bedeutung hat. 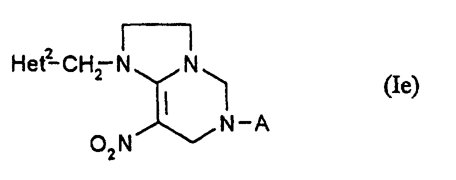 in welcher
- Het²: für gegebenenfalls einfach bis zweifach, gleich oder verschieden durch Chlor, oder Brom substituiertes Thiazolyl steht und
- A: die oben genannte Bedeutung hat. 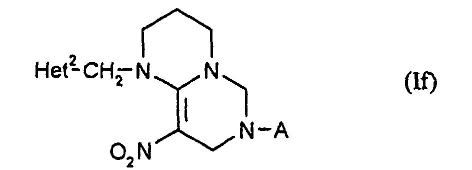 in welcher
- Het²: für gegebenenfalls einfach bis zweifach, gleich oder verschieden durch Fluor, Chlor oder Brom substituiertes Thiazolyl steht und
- A: die oben genannte Bedeutung hat.

Bevorzugte erfindungsgemäße Verbindungen sind auch Stoffgruppen der Formeln (Ia-1), (Ib-1), (Ic-1) und (Id-1): 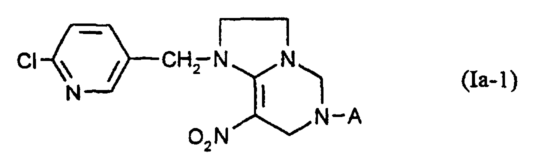 in welcher
- A: für die oben genannten Bedeutungen steht. 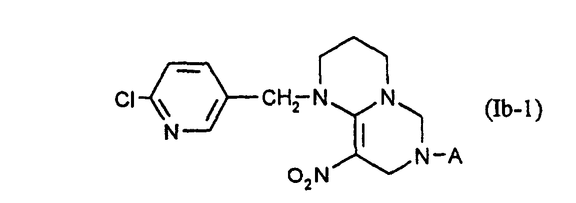 in welcher
- A: für die oben genannten Bedeutungen steht. 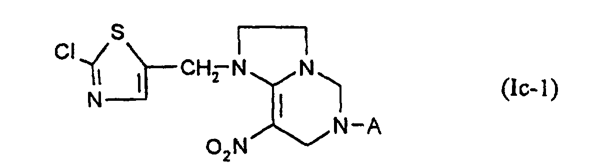 in welcher
- A: für die oben genannten Bedeutungen steht. 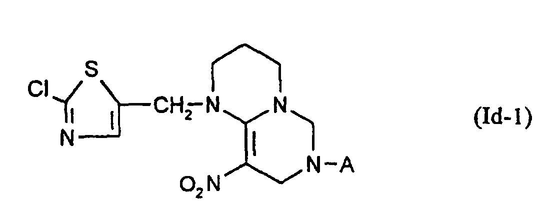 in welcher
- A: für die oben genannten Bedeutungen steht.

Verwendet man zur Durchführung des erfindungsgemäßen Verfahrens (a) beispielsweise 3-(2-Chlorpyridin-5-yl-methyl)-2-nitromethylen-imidazolidin, Glycinmethylester-hydrochlorid und 2 Mol Formaldehyd, so kann der Reaktionsablauf durch das folgende Formelschema skizziert werden:

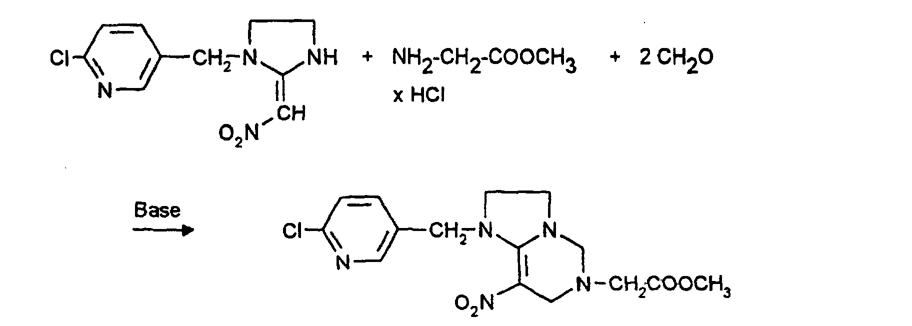

Verwendet man zur Durchführung des erfindungsgemäßen Verfahrens (b) beispielsweise 6,7-Dihydro-6-methoxycarbonylmethyl-8-nitro-(5H)-imidazolidino-[2,3-f]-pyrimidin und 2-Chlor-5-chlormethyl-pyridin, so kann der Reaktionsablauf durch das folgende Formelschema skizziert werden:

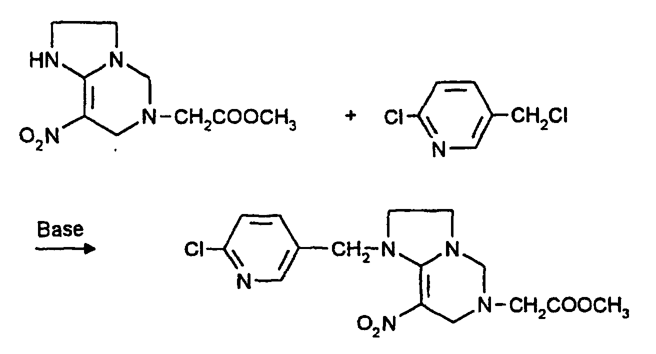

Die beim erfindungsgemäßen Verfahren (a) als Ausgangsstoffe zu verwendenden Nitromethylen-Derivate der Formel (II) sind bekannt (vgl. z.B. EP-A 0 163 855, EP-A 0 192 060, EP-A 0 247 477, EP-A 0 316 843 und EP-A 0 316 845) und/ oder lassen sich nach bekannten Methoden herstellen (vgl. die genannten offengelegten europäischen Anmeldungen).

Werden die Nitromethylen-Derivate in Form ihrer Hydrohalogenide eingesetzt, kommen vorzugsweise die Hydrochloride in Betracht.

Die beim erfindungsgemäßen Verfahren (a) außerdem als Ausgangsstoffe zu verwendenden Amine der Formel (III-a) und (III-b) sind allgemein bekannte Verbindungen der organischen Chemie und/oder können in üblicher Art und Weise erhalten werden.

Die beim erfindungsgemäßen Verfahren (b) als Ausgangsstoffe zu verwendenden Nitropyrimidin-Derivate der Formel (IV) sind noch nicht bekannt. Sie können erhalten werden, indem man Nitromethylen-Derivate der Formel (IIa) (vgl. z.B. EP-A 0 247 477) 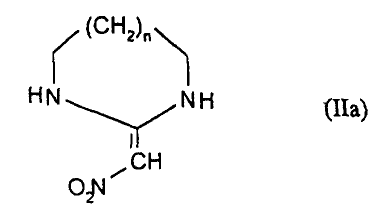 in welcher
- n: die oben angegebene Bedeutung hat,
mit Aminen der Formel (III-a) und (III-b) gemäß Verfahren (a) umsetzt.

Die beim erfindungsgemäßen Verfahren (b) außerdem als Ausgangsstoffe zu verwendenden Verbindungen sind durch die Formel (V) allgemein definiert. L steht vorzugsweise für Chlor, Brom, Iod, Acetoxy, Tosyl oder Mesyl. Die Verbindungen der Formel (V) sind allgemein bekannte Substanzen der organischen Chemie.

Das erfindungsgemäße Verfahren (a) wird vorzugsweise unter Verwendung von Verdünnungsmitteln durchgeführt. Als Verdünnungsmittel kommen dabei Wasser. und für die Umsetzung inerte organische Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligoin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Alkohole, wie Methanol, Ethanol, n-Propanol und Isopropanol. Bevorzugt werden Gemische aus Alkoholen und Wasser eingesezt.

Das erfindungsgemäße Verfahren (a) wird gegebenenfalls in Gegenwart von sauren, nicht oxidierenden Katalysatoren durchgeführt. Besonders bewährt haben sich Halogenwasserstoffsäuren wie Salzsäure und Bromwasserstoffsäure, Phosphorsäure, niedere Carbonsäuren wie Essigsäure und Propionsäure.

Die Reaktionstemperaturen können bei dem erfindungsgemäßen Verfahren (a) in einem größeren Bereich variiert werden. Im allgemeinen arbeitet man bei Temperaturen zweischen -20°C und + 120°C, vorzugsweise bei Temperaturen zwischen 0°C und + 80°C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) setzt man auf 1 Mol Nitromethylen-Derivat der Formel (II), 1 bis 1,5 Mol, vorzugsweise 1 bis 1,2 Mol Amin der Formel (III) und 2 bis 4 Mol, vorzugsweise 2 bis 3 Mol Formaldehyd ein.

Die Amine der Formel (III) können gegebenenfalls als wäßrige Lösungen eingesetzt werden. Bei der Verwendung von gasförmigen Aminen der Formel (III) können diese Verbindungen durch das Gemisch aus Verdünnungsmittel, Verbindungen der Formel (II) und Formaldehyd geleitet werden. Für das erfindungsgemäße Verfahren wird Formaldehyd in wäßriger Lösung eingestzt. Die Reaktionen werden im allgemeinen in einem geeigneten Verdünnungsmittel durchgeführt und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Das erfindungsgemäße Verfahren (b) wird vorzugsweise unter Verwendung von Verdünnungsmitteln durchgeführt. Als Verdünnungsmittel kommen praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- oder Methyl-isobutyl-keton, Ester wie Essigsäuremethylester oder -ethylester, Nitrile wie z.B. Acetonitril oder Propionitril, Amide wie z.B. Dimethylformamid, Dimethylacetamid und N-Methylpyrrolidon sowie Dimethylsulfoxid, Tetramethylensulfon oder Hexamethylenphosphorsäuretriamid.

Als Basen können bei den erfindungsgemäßen Verfahren (a) und (b) alle üblicherweise für derartige Umsetzungen verwendbaren Säurebindemittel eingesetzt werden. Vorzugsweise in Frage kommen Alkalimetall- und Erdalkalimetallhydride, wie Lithium-, Natrium-, Kalium- oder Calciumhydrid, Alkalimetall- oder Erdalkalimetallhydroxide, wie Lithium-, Natrium-, Kalium- oder Calciumhydroxid, Alkalimetall- oder Erdalkalimetallcarbonate oder -hydrogencarbonate, wie Natrium- oder Kaliumcarbonate oder -hydrogencarbonat oder Calciumcarbonat, Alkalimetallacetate, wie Natrium- oder Kaliumacetat, Alkalimetallalkoholate, wie Natrium- oder Kalium-tert.-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) oder 1,4-Diazabicyclo-[2,2,2]-octan (DABCO).

Die Reaktionstemperaturen können beim erfindungsgemäßen Verfahren (b) in einem größeren Bereich variiert werden. Im allgemeinen arbeitet man bei Temperaturen zwischen 0°C und 100°C, vorzugsweise bei Temperaturen zwischen 10°C und 80°C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt nach üblichen Methoden.
Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp.,

Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Spodoptera litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans. Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp.

Die erfindungsgemäßen Verbindungen der Formel (I) zeichnen sich insbesondere durch eine hohe insektizide Wirksamkeit aus.

Sie lassen sich mit besonders gutem Erfolg zur Bekämpfung von pflanzenschädigenden Insekten, wie beispielsweise gegen die Meerrettichblattkäfer-Larven (Phaedon cochlaeriae), die Raupen der Kohlschabe (Plutella maculipennis), die grüne Reiszikade (Nephotettix cinctriceps), die Raupen des Eulenfalters (Spodoptera frugiperda) oder die Pfirsichblattläuse (Mycus persicae) einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid;(E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram,
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

• Auch eine Mischung mit anderen bekannten Wirkstoffen, wie Herbiziden oder mit Düngemitteln und Wachstumsregulatoren ist möglich.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Wemeckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.,

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie
Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis; Xyleborus spec. Tryptodendron spec. Apate monachus, Bostrychus capucins, Heterobostrychus brunneus, Sinoxylon spec. Dinoderus minutus.

Hautflügler wie
Sirex juvencus, Urocerus gigas, Urocerus gigas taignus, Urocerus augur.

Termiten wie
Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze, wie Lepisma saccarina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und- türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs-bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten Insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/ oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise a-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/ oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der obengenannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der Wo 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Die Herstellung und die Verwendung der erfindungsgemäßen Wirkstoffe gehen aus den nachfolgenden Beispielen hervor.

### Herstellungsbeispiele

### Beispiel 1 (nicht erfindungsgemäß)

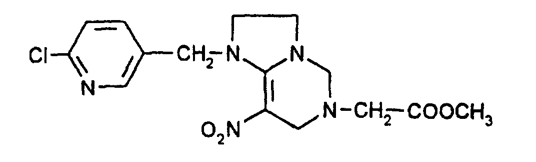

(Verfahren a)
5,0 g (19,6 mmol) 3-(2-Chlorpyridin-5-yl-methyl)-2-nitro-methylen-imidazolidin, 3,0 g (23,9 mmol) Glycinmethylester-hydrochlorid und 3,3 ml Triethylamin werden zusammen mit 3,6 ml 37 %-iger Formaldehyd-Lösung in 30 ml Ethanol gelöst und 5 Stunden unter Rückfluß erhitzt.

Man läßt das Reaktionsgemisch abkühlen und engt durch Abdestillieren des Lösungsmittels im Vakuum ein. Der Rückstand wird mit Wasser verrührt, abgesaugt und getrocknet.

Man erhält 8,9 g 6,7-Dihydro-1-(2-chlorpyridin-5-yl-methyl)-6-methoxycarbonylmethyl-8-nitro-(5H)-imidazolidino-[2,3-f]-pyrimidin vom Schmelzpunkt 168°C.

### Beispiel 2 (nicht erfindungsgemäß)

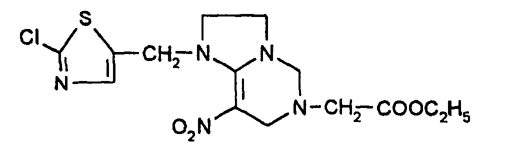

(Verfahren b)
1,5 g (5,86 mmol) 6,7-Dihydro-6-ethoxycarbonyl-methyl-8-(5H)-imidazolidino-[2,3-f]-pyrimidin (Bsp. IV-1) in 20 ml Acetonitril werden mit 1,6 Kaliumcarbonat und 1,0 g (6,0 mmol) 2-Chlor-5-chlormethylthiazol versetzt. Das Reaktionsgemisch wird 6 Stunden bei 40°C gerührt. Danach wird von unlöslichen Bestandteilen filtriert und bis zur Trockne eingedampft. Der Rückstand wird säulenchromatografisch gereinigt.

Man erhält 1,48 g 6,7-Dihydro-1-(2-chlorthiazol-5-yl-methyl)-6-ethoxycarbonylmethyl-8-nitro-(5H)-imidazolidino-[2,3-f]-pyrimidin vom Schmelzpunkt 151°C.

### Herstellung des Ausgangsproduktes

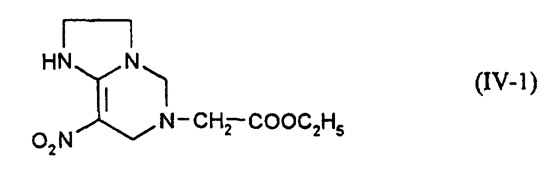

2,0 g (20,2 mmol) 2-Nitromethylen-imidazolidin in 30 ml Ethanol werden mit 2,8 g (20,1 mmol) Glycinethylester-hydrochlorid, 2,7 ml Triethylamin und 4 ml 37 %-iger, wässriger Formaldehydlösung versetzt und 10 Stunden unter Rückfluß erhitzt. Man läßt das Reaktionsgemisch abkühlen und engt durch Abdestillieren des Lösungsmittels ein. Der Rückstand wird zwischen Wasser und Diethylether verteilt, die organische Phase abgetrennt, über Natriumsulfat getrocknet und eingeengt.

Man erhält 2,0 g 6,7-Dihydro-6-ethoxycarbonylmethyl-8-nitro-(5H)-imidazolidino-[2,3-f]-pyrimidin vom Schmelzpunkt 155-158°C.

Analog den Beispielen 1 und 2 sowie gemäß den allgemeinen Angaben zur Herstellung werden die in der nachfolgenden Tabelle 1 aufgeführten Verbindungen der Formel (I) erhalten:

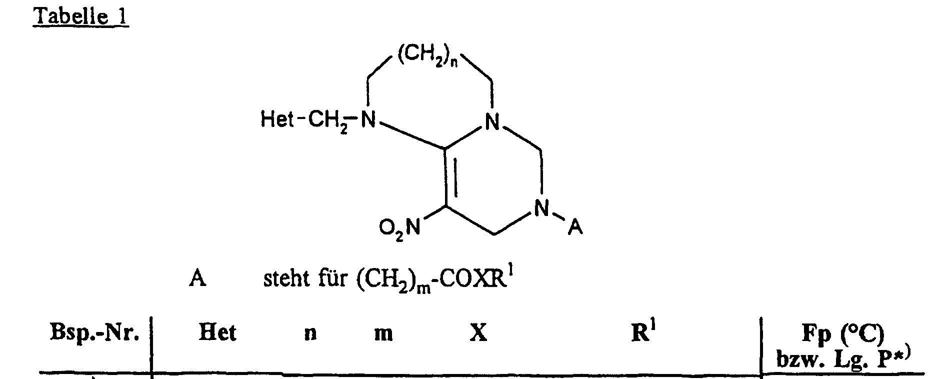

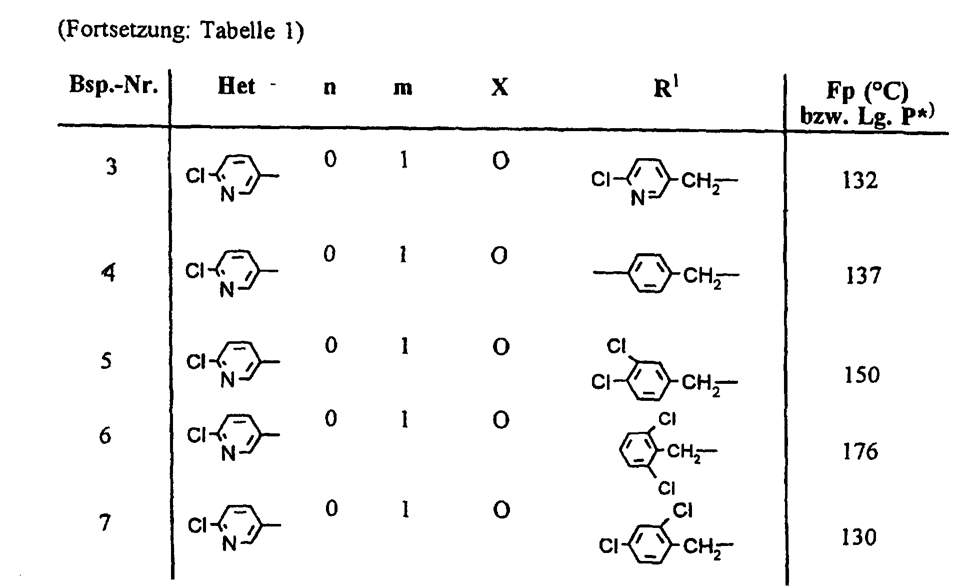

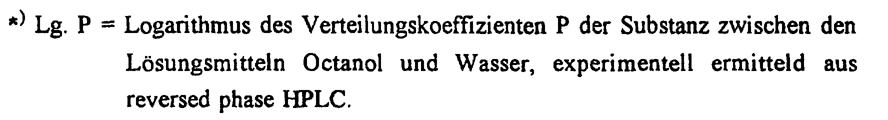

### Anwendungsbeispiele

In den folgenden Anwendungsbeispielen werden die nachstehend aufgeführten Verbindungen als Vergleichssubstanzen eingesetzt:

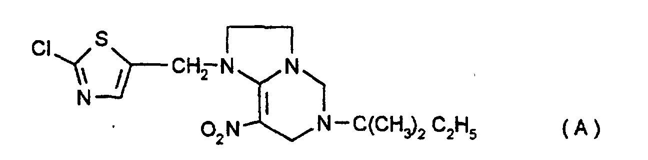

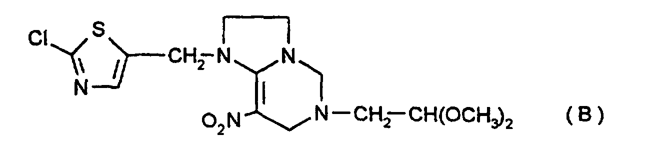

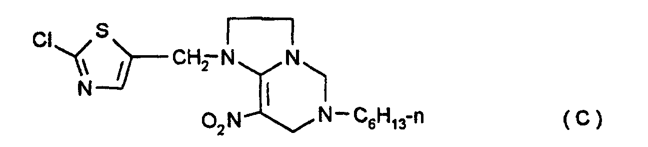

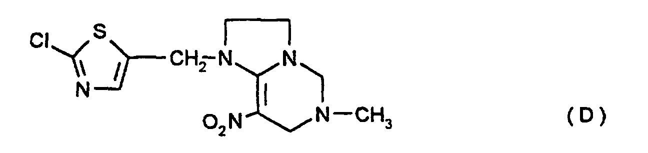

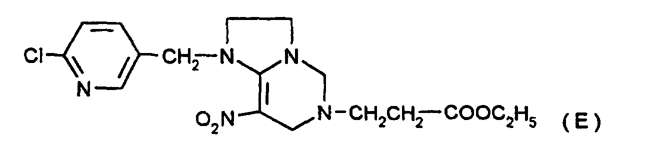

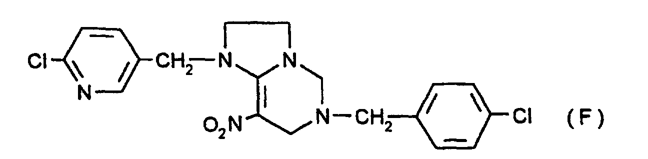

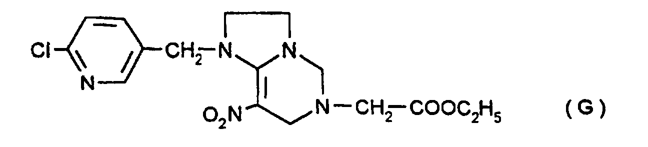

### (Alle Verbindungen bekannt aus EP-A 0 247 477)

### Beispiel A

| **Phaedon-Larven-Test** | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven. (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test bewirkten bei einer beispielhaften Wirkstoffkonzentration von 0,001 % z.B. die Verbindungen der Herstellungsbeispiele 3, 4, 5 und 7 eine Abtötung von 100 % jeweils nach 3 Tagen, während die bekannten Verbindungen: (A) keine Wirkung, (B) lediglich 30 % Abtötung, (C) lediglich 40 % Abtötung und (E) lediglich 20 %Abtötung zeigten.

### Beispiel B

| **Plutella-Test** | | |
|---|---|---|
| Lösungsmittel | 7 Gewichtsteile | Dimethylfomamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test bewirkten bei einer beispielhaften Wirkstoffkonzentration von 0,01 % z.B. die Verbindungen der Herstellungsbeispiele 4, 5, 6 und 7 eine Abtötung von 100 % jeweils nach 3 Tagen, während die bekannte Verbindung (E) keine Wirkung zeigte.

### Beispiel C

| **Spodoptera Frugiperda-Test** | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Spodoptera frugiperda) besetzt, solange die Blätter noch feucht sind.

Nach der gewünschten Zeit wird die in % bestimmt. Dabei bedeutet 100 %, daß alle Raupen abgetötet wurden; 0 % bedeutet, daß keine Raupen abgetötet wurden.

Bei diesem Test bewirkten bei einer beispielhaften Wirkstoffkonzentration von 0,001 % z.B. die Verbindungen der Herstellungsbeispiele 4, 5 und 7 eine Abtötung von 100 % jeweils nach 3 Tagen, während die bekannten Verbindungen (A), (D) und (F) keine Wirkung bzw. (B), (C) und (G) lediglich eine Abtötung von 30 % zeigten.

### Beispiel D

| **Nephotettix-Test** | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test bewirkten bei einer beispielhaften Wirkstoffkonzentration von 0,0001 % z.B. die Verbindungen der Herstellungsbeispiele 3, 4 eine Abtötung von 100 % jeweils nach 6 Tagen, während die bekannte Verbindung (E) lediglich eine Abtötung von 20 % zeigte.

### Beispiel E

| **Myzus-Test** | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man I Gewichtsteil Wirkstoff mit der angegebenen Menge Lösungsmittel und der angegebenen Menge Emulgator und verdünnt das Konzentrat mit Wasser auf die gewunschte Konzentration.

Keimlinge der Dicken Bohne (Vicia faba), die von der Grünen Pfirsichblattlaus (Myzus persicae) befallen sind, werden in die Wirkstoffzubereitung der gewünschten Konzentration getaucht und in eine Plastikdose gelegt.

Nach der gewünschten Zeit wird die Abtötung in Prozent bestimmt. Dabei bedeutet 100 %, daß alle Blattläuse abgetötet wurden; 0 % bedeutet, daß keine Blattläuse abgetötet wurden.

Bei diesem Test bewirkten bei einer beispielhaften Wirkstoffkonzentration von 0,001 % jeweils nach 1 Tag, die bekannten Verbindungen (E) lediglich 10 % Abtötung während (F) keine Wirkung zeigte.

## Patentansprüche

1. Tetrahydropyrimidin-Derivate der Formel (I) 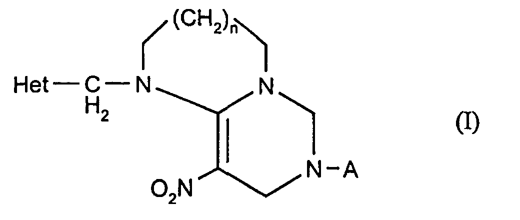 in welcher
A für eine der Gruppierungen (Ia) -(CH₂)ₘ-COXR¹ oder 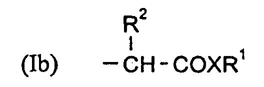
Het für jeweils gegebenenfalls einfach bis zweifach, gleich oder verschieden durch Fluor, Chlor oder Brom substituiertes Pyridyl und Thiazolyl steht,
R¹ für gegebenenfalls einfach bis zweifach, gleich oder verschieden substituiertes Benzyl steht, wobei als Substituenten genannt seien:
Fluor, Chlor, Brom, Cyano, Nitro, Methyl, Ethyl, Methoxy, Methylthio, Trifluormethyl, Trifluormethoxy und Trifluormethylthio;
sowie für jeweils gegebenenfalls einfach bis zweifach, gleich oder verschieden durch Fluor, Chlor oder Brom substituiertes Pyridylmethyl und Thiazolylmethyl,
R² für C₁₋₄-Alkyl oder Benzyl steht,
x für sauerstoff oder die -NH-Gruppierung steht,
m für ganze Zahlen von 1 bis 8 steht und
n für die Zahlen 0 und 1 steht.

2. Verfahren zur Herstellung der Verbindungen der Formel (I) gemäß Anspruch 1, **dadurch gekennzeichnet, daß** man
a) Nitromethylen-Derivate der Formel (II) 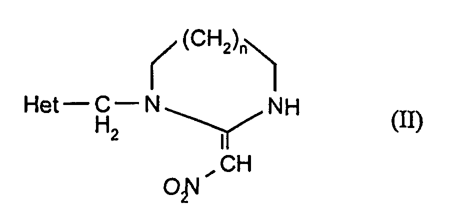 in welcher
Het und n die in Anspruch 1 angegebene Bedeutung haben,
mit Aminen der Formel (IIIa) oder (IIIb)
H₂N―(CH₂)ₘ·CO·X-R¹ (IIIa) 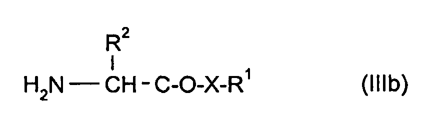
in welcher
R¹, R², X und m die oben angegebene Bedeutung haben, gegebenenfalls in Form ihrer Hydrohalogenide in Gegenwart von mindestens der zweifachen molaren Menge Formaldehyd, gegebenenfalls in Gegenwart eines sauren Katalysators und gegebenenfalls in Gegenwart eines Verdünnungsmittels sowie gegebenenfalls in Gegenwart einer Base umsetzt;
oder
b) Nitropyrimidin-Derivate der Formel (IV) 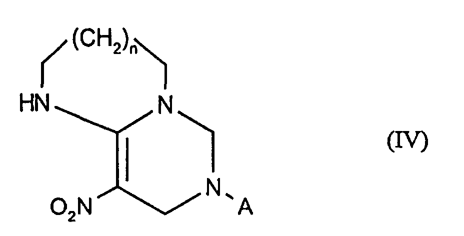
in welcher
A und n die in Anspruch 1 angegebene Bedeutung haben,
mit Verbindungen der Formel (V)
Het-CH₂-L (V)
in welcher
Het die oben angegebene Bedeutung hat und
L für eine anionische Abgangsgruppe steht, in Gegenwart einer Base und gegebenenfalls in Gegenwart eines Verdünnungsmittels umsetzt.

3. Nitropyrimidin-Derivate der Formel (IV) 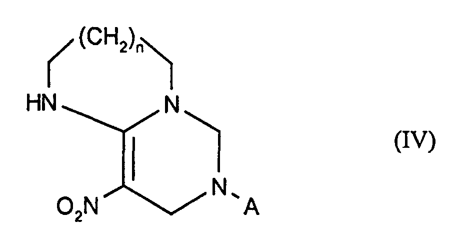 in welcher
A, und n die in Anspruch 1 angegebenen Bedeutungen haben.

4. Verfahren zur Herstellung der Nitropyrimidin-Derivate der Formel (IV) gemäß Anspruch 3, **dadurch gekennzeichnet, daß** man Nitromethylen-Derivate der Formel (IIa) 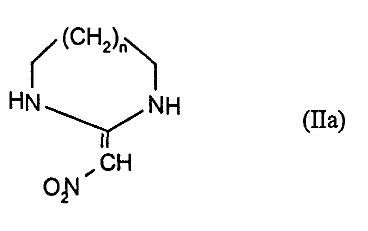 in welcher
n für 0 oder 1 steht,
mit Aminen der Formel (IIIa) oder (IIIb)
H₂N―(CH₂)ₘ·CO·X-R¹ (IIIa) 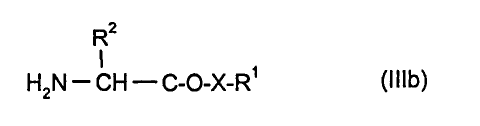
in welcher
R¹, R², X und m die in Anspruch 1 angegebene Bedeutung haben,
gegebenenfalls in Form ihrer Hydrohalogenide in Gegenwart von mindestens der zweifachen molaren Menge Formaldehyd, gegebenenfalls in Gegenwart eines sauren Katalysators und gegebenenfalls in Gegenwart eines Verdünnungsmittels sowie gegebenenfalls in Gegenwart einer Base umsetzt.

5. Schädlingsbekämpfungsmittel, **gekennzeichnet durch** einen Gehalt an mindestens einem Tetrahydropyrimidin der Formel (I), gemäß Anspruch 1.

6. Verwendung von Tetrahydropyrimidinen der Formel (I) gemäß Anspruch 1 zur Bekämpfung von Schädlingen.

7. Verfahren zur Bekämpfung von Schädlingen, **dadurch gekennzeichnet, daß** man Tetrahydropyrimidine der Formel (I) gemäß Anspruch 1 auf Schädlinge und/oder ihren Lebensraum einwirken läßt.

8. Verfahren zur Herstellung von Schädlingsbekämpfungsmitteln, **dadurch gekennzeichnet, daß** man Tetrahydropyrimidine der Formel (I) gemäß Anspruch 1 mit Streckmitteln und/oder oberflächenaktiven Mitteln vermischt.

9. Verwendung von Tetrahydropyrimidinen der Formel (I) gemäß Anspruch 1 zur Herstellung von Schädlingsbekämpfungsmitteln.

## Claims

1. Tetrahydropyrimidine derivatives of the formula (I) 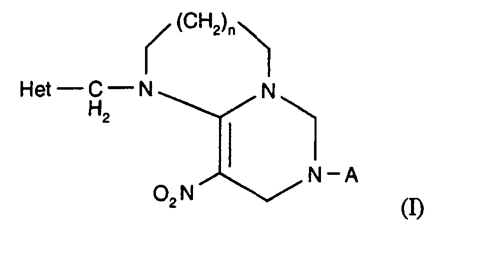 in which
A represents one of the groupings (Ia) -(CH₂)ₘ-COXR¹ or (Ib) 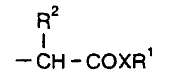
Het represents pyridyl and thiazolyl, each of which is optionally substituted by one to two identical or different substituents from the group consisting of fluorine, chlorine and bromine,
R¹ represents benzyl which is optionally substituted by one to two identical or different substituents, suitable substituents being:
fluorine, chlorine, bromine, cyano, nitro, methyl, ethyl, methoxy, methylthio, trifluoromethyl, trifluoromethoxy and trifluoromethylthio; or pyridylmethyl and thiazolylmethyl, each of which is optionally substituted by one to two identical or different substituents from the group consisting of fluorine, chlorine or bromine,
R2 represents C₁-C₄-alkyl or benzyl,
x represents oxygen or the -NH- grouping,
m represents integers from 1 to 8 and
n represents the numbers 0 and 1.

2. Process for preparing the compounds of the formula (I) according to Claim 1,
**characterized in that**
a) nitromethylene derivatives of the formula (II) 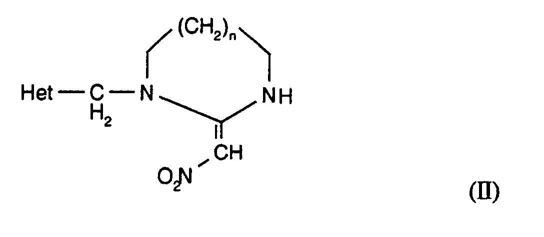 in which
Het and n are as defined in Claim 1
are reacted with amines of the formula (IIIa) or (IIIb)
H₂N―(CH₂)ₘ-CO-X-R¹ (IIIa) 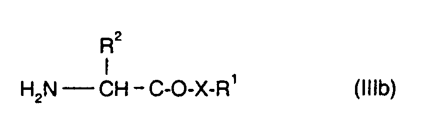
in which
R¹, R², X and m are as defined above,
optionally in the form of their hydrohalides in the presence of at least twice the molar amount of formaldehyde, if appropriate in the presence of an acid catalyst and if appropriate in the presence of a diluent and if appropriate in the presence of a base;
or
b) nitropyrimidine derivatives of the formula (IV) 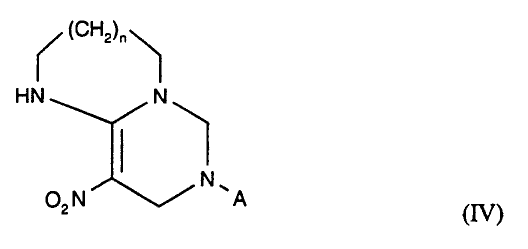 in which
A and n are as defined in Claim 1,
are reacted with compounds of the formula (V)
Het-CH₂-L (V)
in which
Het is as defined above and
L represents an anionic leaving group,
in the presence of a base and, if appropriate, in the presence of a diluent.

3. Nitropyrimidine derivatives of the formula (IV) 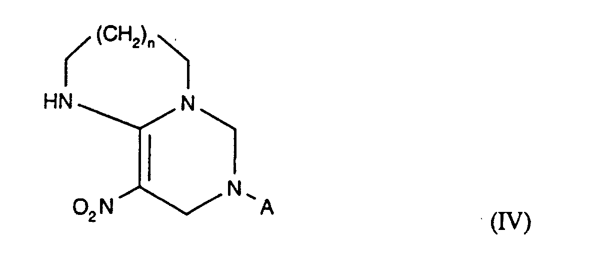 in which
A and n are as defined in Claim 1.

4. Process for preparing the nitropyrimidine derivatives of the formula (IV) according to Claim 3, **characterized in that** nitromethylene derivatives of the formula (IIa) 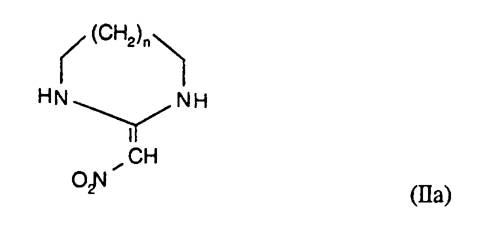 in which
n is 0 or 1,
are reacted with amines of the formula (IIIa) or (IIIb)
H₂N―(CH₂)ₘ-CO-X-R¹ (IIIa) 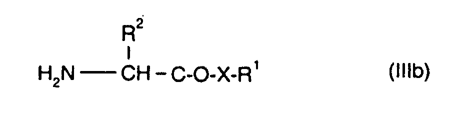
in which
R¹, R², X and m are as defined in Claim 1,
optionally in the form of their hydrohalides in the presence of at least twice the molar amount of formaldehyde, if appropriate in the presence of an acid catalyst and if appropriate in the presence of a diluent and if appropriate in the presence of a base.

5. Pesticides, **characterized in that** they comprise at least one tetrahydropyrimidine of the formula (I) according to Claim 1.

6. Use of tetrahydropyrimidines of the formula (I) according to Claim 1 for controlling pests.

7. Method for controlling pests, **characterized in that** tetrahydropyrimidines of the formula (I) according to Claim 1 are allowed to act on the pests and/or their habitat.

8. Process for preparing pesticides, **characterized in that** tetrahydropyrimidines of the formula (I) according to Claim 1 are mixed with extenders and/or surfaceactive agents.

9. Use of tetrahydropyrimidines of the formula (I) according to Claim 1 for preparing pesticides.

## Revendications

1. Dérivés de tétrahydropyrimidine de formule (I) 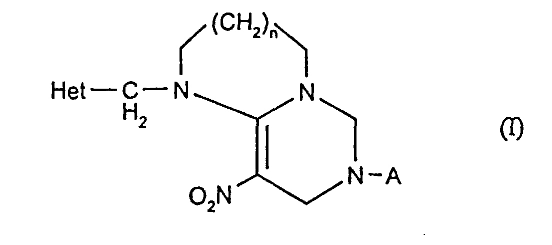 dans laquelle
A représente l'un des groupements (Ia)-(CH₂)ₘ-COXR¹ ou 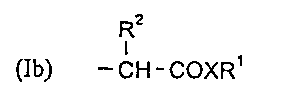
Het représente un groupe pyridyle ou un groupe thiazolyle dont chacun porte, le cas échéant, un ou deux substituants fluor, chlore ou brome identiques ou différents,
R1 représente un groupe benzyle portant éventuellement un ou deux substituants identiques ou différents, les substituants pouvant être les suivants :
fluor, chlore, brome, cyano, nitro, méthyle, éthyle, méthoxy, méthylthio, trifluorométhyle, trifluorométhoxy et trifluorométhylthio ;
ainsi qu'un groupe pyridylméthyle et un groupe thiazolylméthyle dont chacun peut porter un ou deux substituants, identiques ou différents, fluor, chlore ou brome,
R2 est un groupe alkyle en C₁ à C₄ ou le groupe benzyle,
x représente l'oxygène ou le groupement -NH-,
m représente des nombres entiers de 1 à 8 et
n représente les nombres 0 et 1.

2. Procédé de production des composés de formule (I) suivant la revendication 1, **caractérisé en ce que** :
a) on fait réagir des dérivés nitrométhyléniques de formule (II) 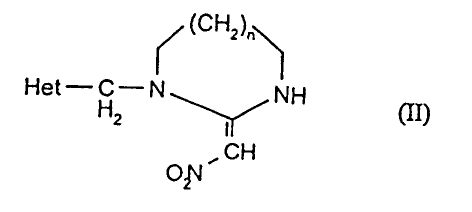 dans laquelle
Het et n ont la définition indiquée dans la revendication 1,
avec des amines de formule (IIIa) ou (IIIb)
H₂N―(CH₂)ₘ·CO·X-R¹ (IIIa) 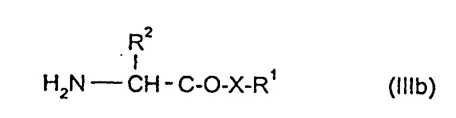
dans laquelle
R¹, R², X et m ont la définition indiquée ci-dessus, le cas échéant sous forme de leurs halogénhydrates en présence d'au moins la double quantité molaire de formaldéhyde, éventuellement en présence d'un catalyseur acide et en présence éventuelle d'un diluant ainsi que, le cas échéant, en présence d'une base ;
ou bien
b) on fait réagir des dérivés de nitropyrimidine de formule (IV) 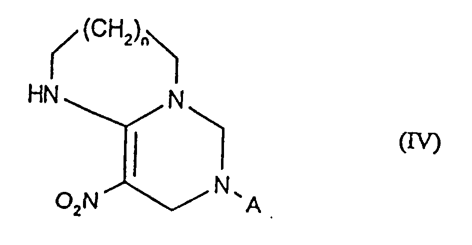
dans laquelle
A et n ont la définition indiquée dans la revendication 1,
avec des composés de formule (V)
Het-CH₂-L (V)
dans laquelle
Het a la définition indiquée ci-dessus et
L représente un groupe anionique partant,
en présence d'une base et en présence éventuelle d'un diluant.

3. Dérivés de nitropyrimidine de formule (IV) 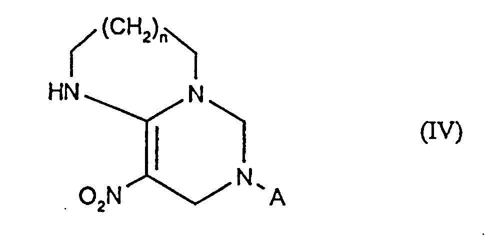 dans laquelle
A et n ont les définitions indiquées dans la revendication 1.

4. Procédé de production des dérivés de nitropyrimidine de formule (IV) suivant la revendication 3, **caractérisé en ce qu'**on fait réagir des dérivés nitrométhyléniques de formule (IIa) 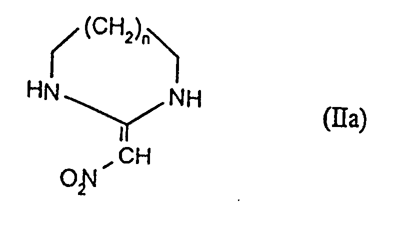 dans laguelle
n a la valeur 0 ou 1,
avec des amines de formule (IIIa) ou (IIIb)
H₂N―(CH₂)ₘ·CO·X-R¹ (IIIa) 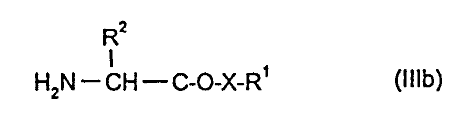
dans laquelle
R¹, R², X et m ont la définition indiquée dans la revendication 1,
éventuellement sous forme de leurs halogénhydrates en présence d'au moins la double quantité molaire de formaldéhyde, éventuellement en présence d'un catalyseur acide et éventuellement en présence d'un diluant ainsi que, le cas échéant, en présence d'une base.

5. Compositions pesticides, **caractérisées par** une teneur en au moins une tétrahydropyrimidine de formule (I) suivant la revendication 1.

6. Utilisation de tétrahydropyrimidines de formule (I) suivant la revendication 1 pour la lutte contre des parasites.

7. Procédé de lutte contre des parasites, **caractérisé en ce qu'**on fait agir des tétrahydropyrimidines de formule (I) suivant la revendication 1 sur les parasites et/ou sur leur milieu.

8. Procédé de préparation de compositions pesticides, **caractérisé en ce qu'**on mélange des tétrahydropyrimidines de formule (I) suivant la revendication 1 avec des diluants et/ou des agents tensio-actifs.

9. Utilisation de tétrahydropyrimidines de formule (I) suivant la revendication 1 pour la préparation de compositions pesticides.
